(19) Europäisches Patentamt
European Patent Office
Office européen des brevets

(11) Publication number: 0 000 167 A1

# (12) EUROPEAN PATENT APPLICATION

(21) Application number: 78100190.4

(22) Date of filing: 19.06.78

(51) Int. Cl.$^2$: C 07 D 211/78, C 07 D 211/62, A 61 K 31/435

(30) Priority: 20.06.77 GB 25740/77

(43) Date of publication of application: 10.01.79 Bulletin 79/1

(84) Designated Contracting States: BE CH DE FR GB LU NL SE

(71) Applicant: Krogsgaard-Larsen, Povl,
Elmevej 25 Blovstroed,
DK-3450 Alleroed (DK)

(84) Designated Contracting States: BE CH DE FR GB LU NL SE

(71) Applicant: Falch, Erik,
Olgasvej 31,
DE-2950 Vedbaek (DK)

(84) Designated Contracting States: BE CH DE FR GB LU NL SE

(72) Inventor: Krogsgaard-Larsen, Povl,
Elmevej 25 Blovstroed,
DK-3450 Alleroed (DK)

(72) Inventor: Falch, Erik,
Olgasvej 31,
DK-2950 Vedbaek (DK)

(74) Representative: Patentanwälte Dipl.-Ing. A. Grünecker, Dr.-Ing. H. Kinkeldey, Dr.-Ing. H Stockmair,; Dr. rer. nat. K.Schumann, Dipl.-Ing. P. Jacob, Dr. rer. nat. G. Bezold
Maximilianstrasses 43,
D-8000 München 22 (DE)

## (54) 1,2,3,6-Tetrahydroisonicotinic acid and derivatives thereof, methods and starting products for their preparation, and pharmaceutical compositions containing them.

(57) 1,2,3,6-Tetrahydroisonicotinic acid and derivatives thereof, methods and starting products for their preparation, and pharmaceutical compositions containing them. Compounds of formula

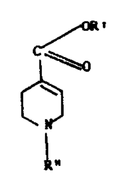

is which R" is hydrogen, acetyl or a group

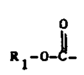

wherein $R_1$ is C1-8 alkyl; phenyl; substituted phenyl, phenylalkyl in which the phenyl group may be substituted. R' is hydrogen; C1-8 alkyl; phenyl; substituted phenyl, phenylalkyl, substituted phenylalkyl or idanyl; or R' is a group
wherein $R_2$ and $R_3$ are hydrogen; C1-6 alkyl; or phenylalkyl and $R_4$ designates C1-8 alkyl; phenyl; substituted phenyl or phenylalkyl and salts thereof.

Intermediates for preparing (I) are

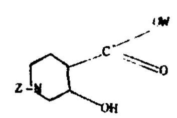

in which Z is hydrogen or a protecting group and W is hydrogen or a readily removable group and salts thereof which are subjected to dehydration and

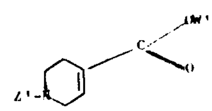

in which Z' and W' have the same meaning as Z and W and salts thereof, which are reacted with appropriate reagents.

The compounds (I) exhibit γ-aminobutyric acid related activity and are useful as active ingredients in pharmaceutical compositions. They may additionally contain a minor tranquilizer or a neuroleptic.

EP 0 000 167 A1

0000167

CYCLIC AMINO ACIDS.

**TITLE MODIFIED**
**see front page**

The present invention relates to compounds showing GABA--related activity.

GABA (gamma-aminobutyric acid) is known to be a neurotransmitter in the central nervous system (CNS) in mammals. GABA is found predominantly in the brain where it is a dominant inhibitory transmitter (Curtis, D.R. and Johnston, G.A.R., Ergebn. Physiol., 1974, 69, 97 - 188).

It has been reported (Arzneimittelforschung, 1968, 18, 311 - 315) that muscimol of the formula

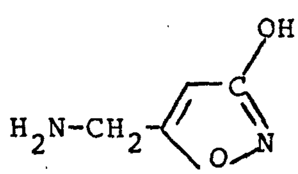

(a substance found in fly amanita (Amanita muscaria)) has various interesting pharmacological properties and especially shows an inhibition of motoric functions. Later, it was reported that muscimol is a very potent GABA agonist with respect to bicuculline-sensitive postsynaptic receptors

(Johnston et al., Biochem. Pharmacol., 1968, 17, 2488, and Curtis et al., Brain Res., 1971, 32, 69 - 96), but it also shows activity as an inhibitor of the high affinity uptake of GABA in rat brain slices (Johnston, Psychopharmacologia, 1971, 22, 230 - 233). Reduced function in the GABA system is believed to be related to the etiology of parkinsonism, epilepsy, Huntington's chorea (Thomas N. Chase and Judith R. Walters, GABA in Nervous System Function, edited by E. Roberts, T.N. Chase and D.B. Tower, Raven Press, New York, 1976, 497 - 513), and schizophrenia, and administration of agents influencing the GABA system is therefore under consideration and research for the therapeutical treatment of such GABA system malfunction-related diseases. It is also under consideration to administer agents influencing the GABA system against diseases in which malfunctions of the pituitary hormones are involved, and it is, furthermore, contemplated that such agents may be useful against artereoschlerotic diseases in the brain where a vasodilatation is desired. However, unfortunately, muscimol has poisoneous effects, such as narcotic effects (derealisation and depersonalisation), and the difference between the effective dose and the toxic dose of muscimol is very small (Arzneimittelforschung, 1968, 18, 311 - 315), which may limit or prevent the therapeutic use of muscimol. Furthermore, it would be highly desirable to provide a substance having a more specific GABA activity than muscimol which, as mentioned above, shows considerable GABA-uptake inhibitor activity in addition to its GABA agonist activity. In an attempt to establish a structure/activity relation, various muscimol-analogues or muscimol-like substances have been synthesized and tested (P. Krogsgaard-

Larsen et al., Journal of Neurochemistry, 1975, 25, 797 - 802 and 803 - 809 . However, none of the compounds tested showed a GABA agonist activity of the same potency as that of muscimol.

The present invention relates to novel compounds showing GABA--related activity, to salts thereof with acids or bases, and to pharmaceutical compositions containing compounds having GABA--related activity or salts thereof as active ingredient. Moreover, the present invention relates to methods for the preparation of the novel compounds and salts thereof and to a method for the treatment of neurological and psychiatrical disorders, such as epilepsy, parkinsonism, schizophrenia and Huntington's chorea, or diseases in which malfunctions of the pituitary hormones are involved, or artereoschlerotic diseases in the brain where a vasodilatation is desired, by administering a therapeutically effective amount of the compounds or salts thereof to a living animal body including human beings.

According to the present invention, it has been found that the novel compound of the formula Ia

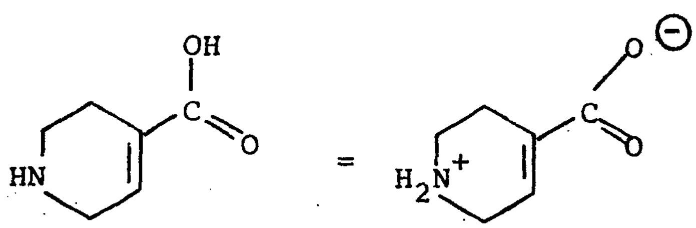

Ia

1,2,3,6-tetrahydroisonicotinic acid ("isoguvacine")

is a specific and very potent GABA receptor agonist, vide the section "Test Results" below. A comparison of the formula Ia and GABA shows that GABA is incorporated as a structural element of the molecule of compound Ia. However, zwitterions like the amino acid Ia may not be able to pass the blood-brain barrier, which means that for practical administration, it is often preferred to convert it into a form which will permit the compound to pass the blood-brain barrier and which thereafter will be decomposed in situ in the brain to yield the parent compound.

Recent investigations seem to indicate that GABA receptor agonists unable to penetrate the blood-brain barrier interact directly with the anterior pituitary after systemic administration and inhibit secretion of prolactin. Consequently, systemic administration of compound Ia is expected to decrease prolactin secretion. An undesired effect observed after treatment of patients with the traditional neuroleptics such as butyrophenones and phenothiazines is an increased secretion of prolactin. The side effect of treatment with neuroleptics might be avoided by treatment of patients with combinations of compound Ia and neuroleptic drugs. Thus, the invention also relates to the use of compound Ia in combination with neuroleptic drugs such as butyrophenones and phenothiazines and to the use of Ia in pituitary malfunction-related diseases in humans and animals in which decrease of prolactin secretion is desirable.

On this background, the present invention also provides novel compounds comprising compound Ia and derivatives thereof in a "prodrug" or "transportation" form that is compounds which will pass the blood-brain barrier and which thereafter will be decomposed in situ in the brain to yield the parent compound (Ia), in particular compounds of the general formula I

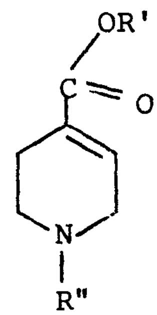

I

in which R" is hydrogen, acetyl or a group of the general formula II

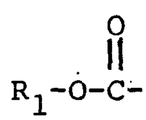

II

wherein $R_1$ is $C_{1-8}$ alkyl; phenyl; phenyl substituted in the 4-position with halogen, lower alkoxy, or lower alkyl; or phenylalkyl such as benzyl or phenylethyl in which the phenyl group may be substituted in the 4-position with halogen, lower alkoxy, or lower alkyl; and R' is hydrogen; $C_{1-8}$ alkyl; phenyl; phenyl substituted in 4-position with halogen, lower alkoxy, lower alkyl or hydroxy; phenylalkyl such as benzyl or phenylethyl; or phenylalkyl substituted in the 4-position of the phenyl moiety with halogen, lower alkoxy, lower alkyl or hydroxy; or indanyl; or R' is a group of the general formula III

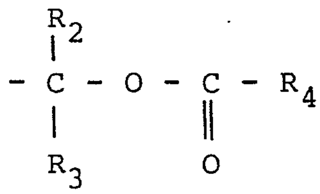

III

wherein $R_2$ and $R_3$ are the same or different and each designate hydrogen; $C_{1-6}$ alkyl; or phenylalkyl such as benzyl or phenylethyl; and $R_4$ designates $C_{1-8}$ alkyl; phenyl; phenyl substituted in the 4-position with halogen, lower alkoxy or lower alkyl; or phenylalkyl such as benzyl or phenylethyl; and salts thereof, with the proviso that when R" is hydrogen, R' is different from ethyl. Among the compounds of the general formula I, compounds in which at least one of R' and R" is hydrogen are preferred for therapeutical administration, as salts; the compounds in which both R' and R" are different from hydrogen tend to be oily substances.

In the present specification, "lower alkyl" and "lower alkoxy" designate such groups containing 1 - 4 carbon atoms.

The compound excluded through the above proviso, that is, the compound of the formula Ib

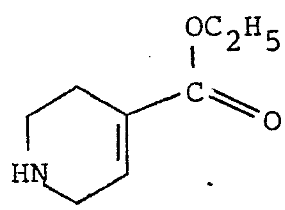

Ib

is known from German Offenlegungsschrift No. 2,221,770, and from Liebigs Ann. Chem., 1972, 764, 21 - 27. In the Liebig article, the compound Ib and analogues having the formula Ic

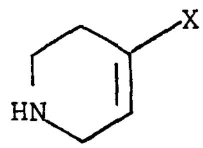

Ic

in which X is cyano or a group $-(CH_2)_n-COOR_5$ in which n is an integer from 0 to 5, and $R_5$ is an optionally substituted alkyl or aryl group, are stated to be valuable intermediates for the preparation of pharmaceutically active substances, especially chloretics, but neither the Liebig article nor the Offenlegungsschrift contain any indication whatsoever of any GABA-related activity of any of these compounds.

Among the compounds of the general formula I, in which R' is one of the hydrocarbon groups stated above are:

methyl 1,2,3,6-tetrahydroisonicotinate,
propyl 1,2,3,6-tetrahydroisonicotinate,
isopropyl 1,2,3,6-tetrahydroisonicotinate,
butyl 1,2,3,6-tetrahydroisonicotinate,
tert.butyl 1,2,3,6-tetrahydroisonicotinate,
phenyl 1,2,3,6-tetrahydroisonicotinate,
4-chlorophenyl 1,2,3,6-tetrahydroisonicotinate,
4-methoxyphenyl 1,2,3,6-tetrahydroisonicotinate,

benzyl 1,2,3,6-tetrahydroisonicotinate,
4-hydroxyphenyl 1,2,3,6-tetrahydroisonicotinate,
and acid addition salts thereof.

Examples of compounds of the general formula I in which R' is hydrogen, and R" is acetyl or a group of the general formula II are:

1-acetyl-1,2,3,6-tetrahydroisonicotinic acid,
1-methoxycarbonyl-1,2,3,6-tetrahydroisonicotinic acid,
1-ethoxycarbonyl-1,2,3,6-tetrahydroisonicotinic acid,
1-propoxycarbonyl-1,2,3,6-tetrahydroisonicotinic acid,
1-butyloxycarbonyl-1,2,3,6-tetrahydroisonicotinic acid,
1-tert.butyloxycarbonyl-1,2,3,6-tetrahydroisonicotinic acid,
1-phenoxycarbonyl-1,2,3,6-tetrahydroisonicotinic acid,
1-(4-chlorophenoxycarbonyl)-1,2,3,6-tetrahydroisonicotinic acid,
1-benzyloxycarbonyl-1,2,3,6-tetrahydroisonicotinic acid,
1-(4-methoxyphenoxycarbonyl)-1,2,3,6-tetrahydroisonicotinic acid,
1-(4-methylphenoxycarbonyl)-1,2,3,6-tetrahydroisonicotinic acid,
and salts thereof with bases.

Examples of compounds of the general formula I in which R' is a group of the general formula III, which compounds constitute preferred compounds of the general formula I, are:

acetoxymethyl 1,2,3,6-tetrahydroisonicotinate,
propionyloxymethyl 1,2,3,6-tetrahydroisonicotinate,
butyryloxymethyl 1,2,3,6-tetrahydroisonicotinate,
isobutyryloxymethyl 1,2,3,6-tetrahydroisonicotinate,

pivaloyloxymethyl 1,2,3,6-tetrahydroisonicotinate,
2-ethylbutyryloxymethyl 1,2,3,6-tetrahydroisonicotinate,
benzoyloxymethyl 1,2,3,6-tetrahydroisonicotinate,
4-chlorobenzoyloxymethyl 1,2,3,6-tetrahydroisonicotinate,
4-methoxybenzoyloxymethyl 1,2,3,6-tetrahydroisonicotinate,
phenylacetoxymethyl 1,2,3,6-tetrahydroisonicotinate,
1-(acetoxy)ethyl 1,2,3,6-tetrahydroisonicotinate,
1-(pivaloyloxy)ethyl 1,2,3,6-tetrahydroisonicotinate,
1-acetoxy-1-methylethyl 1,2,3,6-tetrahydroisonicotinate,
1-pivaloyloxy-1-methylethyl 1,2,3,6-tetrahydroisonicotinate,
1-acetoxy-2-phenylethyl 1,2,3,6-tetrahydroisonicotinate,
1-pivaloyloxy-2-phenylethyl 1,2,3,6-tetrahydroisonicotinate,
and acid addition salts thereof.

Examples of salts of the compounds of formula I in which R" is hydrogen, are acid addition salts thereof, such as pharmaceutically acceptable salts with inorganic acids, e.g. hydrochloric, hydrobromic, nitric, sulfuric, phosphoric acids and the like, or with organic acids, such as organic carboxylic acids, e.g. acetic, propionic, glycolic, malonic, succinic, maleic, fumaric, malic, tartaric, citric, glucuronic, benzoic, pamoic acid and the like, or organic sulfonic acids, e.g. methane sulfonic, ethane sulfonic, benzene sulfonic, toluene sulfonic acid and the like, which salts may be prepared by procedures known per se, e.g. by adding the acid in question to the base, preferably in a solvent. When R" is different from hydrogen, the compounds of formula I may form pharmaceutically acceptable salts with bases, such as metal salts, e.g. sodium, potassium, calcium or aluminum salts, and ammonium and substituted ammonium salts, e.g. salts of amines

such as triethylamine, triethanolamine, ethylpiperidine, procaine, dibenzylamine, and the like.

TEST RESULTS.

Affinity Binding Experiments.

In order to study the interactions of compound Ia with the central GABA receptors in vitro, compound Ia was tested in affinity binding experiments. The affinity binding (sodium-independent binding) of GABA to membranes isolated from rat brains was studied as described by Enna, S.J. and Snyder, S.H., Brain Res., 1975, 100, 81 - 97. $IC_{50}$ values, inhibitor concentrations causing 50% inhibition of GABA binding, were determined.

| Inhibitor | $IC_{50}$ value |
|---|---|
| Muscimol | 0.024 ± 0.003 μM |
| Ia | 0.015 ± 0.001 μM *) |

*) In earlier studies 1.4 ± 0.1 μM was found. The value stated (0.015 ± 0.001 μM) is based on studies of 5 different concentrations of Ia, each determined in triplicate, and the stated $IC_{50}$ value is calculated by log-probit analysis. The difference between the two $IC_{50}$ values determined for Ia is the result of the development of an improved technique for the preparation of rat brain membranes.

Microelectrophoretic Experiments.

In order to study the interactions of compound Ia with the central GABA receptors in vivo, compound Ia was tested in microelectrophoretic experiments. Experiments were performed on lumbar dorsal horn interneurones and Renshaw cells of cats anaesthetized with pentobarbitone sodium. The approximate potency of the depressant actions of the compound was assessed relative to that of GABA on the basis of electrophoretic currents required to produce equal and submaximal inhibitions of the firing of the central neurones. The inhibitory action of Ia on central neurones was antagonized by the specific GABA antagonist bicuculline methochloride (BMC).

| Compound | Potency relative to that of GABA | Reversible antagonism by BMC |
|---|---|---|
| GABA | + + + | yes |
| Ia | + + + + | yes |

The compound Ia did not interact with the GABA uptake system at concentrations of $5 \times 10^{-4}$ M, and did not interact with the GABA metabolizing enzymes GABA:2-oxo-glutarate aminotransferase and L-glutamate 1-carboxylase at concentrations of $10^{-3}$ M.

Based on the above-mentioned experiments, compound Ia is a specific and very potent GABA receptor agonist.

Pharmacological Results in Mice.

Preliminary pharmacological results of tests with the hydrobromide of the compound of the formula I in which R' = $CH_3$ and R" = H, "isoguvacine methylester hydrobromide" (ISM), and the hydrobromide of the compound of the formula I in which R' has the formula III in which $R_4$ is tert.butyl, and $R_2$, $R_3$, and R" are hydrogen, "isoguvacine pivaloyloxymethylester hydrobromide" (ISP), in doses of 160 mg/kg i.p.:

1. ISM and ISP antagonize/enhance the latency time of isoniazide-induced convulsions.

2. ISM restrains morphine-induced hypermotility in minor degree. ISP totally restrains morphine-induced hypermotility for 165 minutes (it is not yet established if ISP in itself has a sedative effect).

The compounds of the invention of the general formula I may be prepared via the acid Ia which, in itself, is suitably prepared as shown in Reaction Schemes I and II:

REACTION SCHEME I:

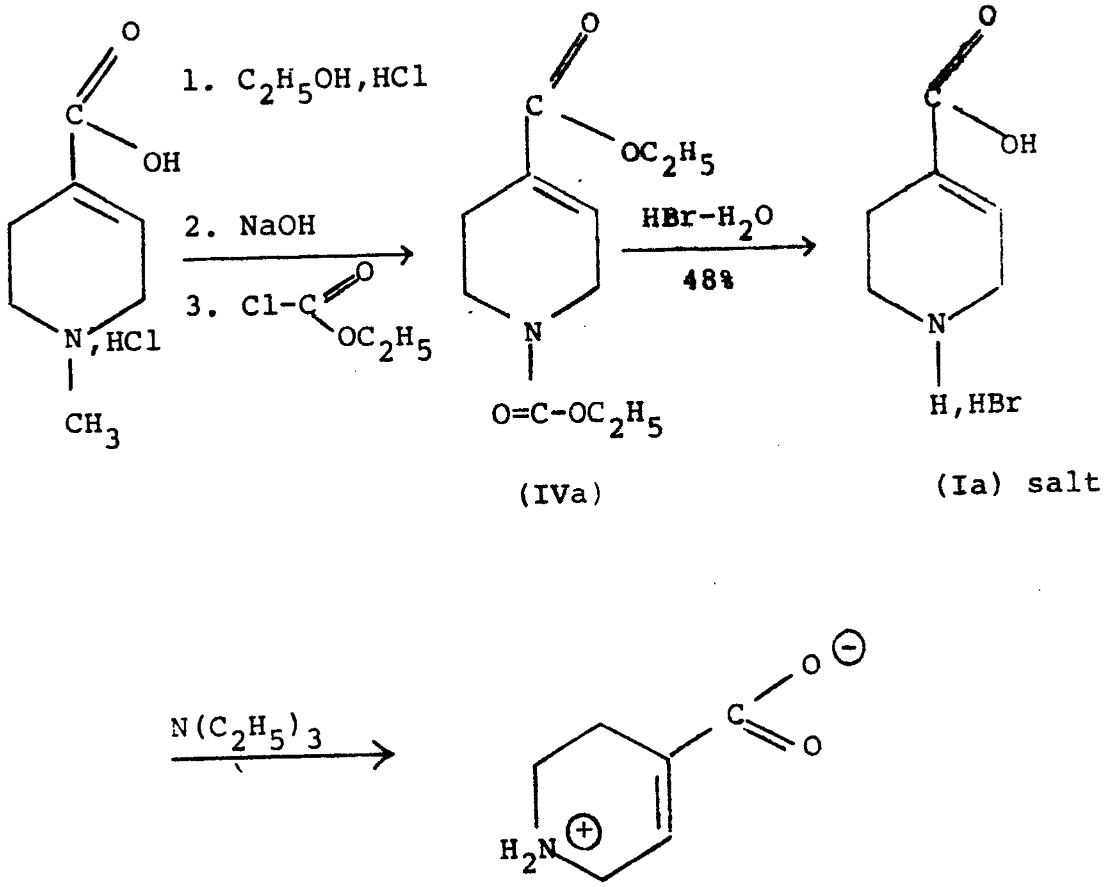


(IVa)

(Ia) salt

(Ia)

REACTION SCHEME II:

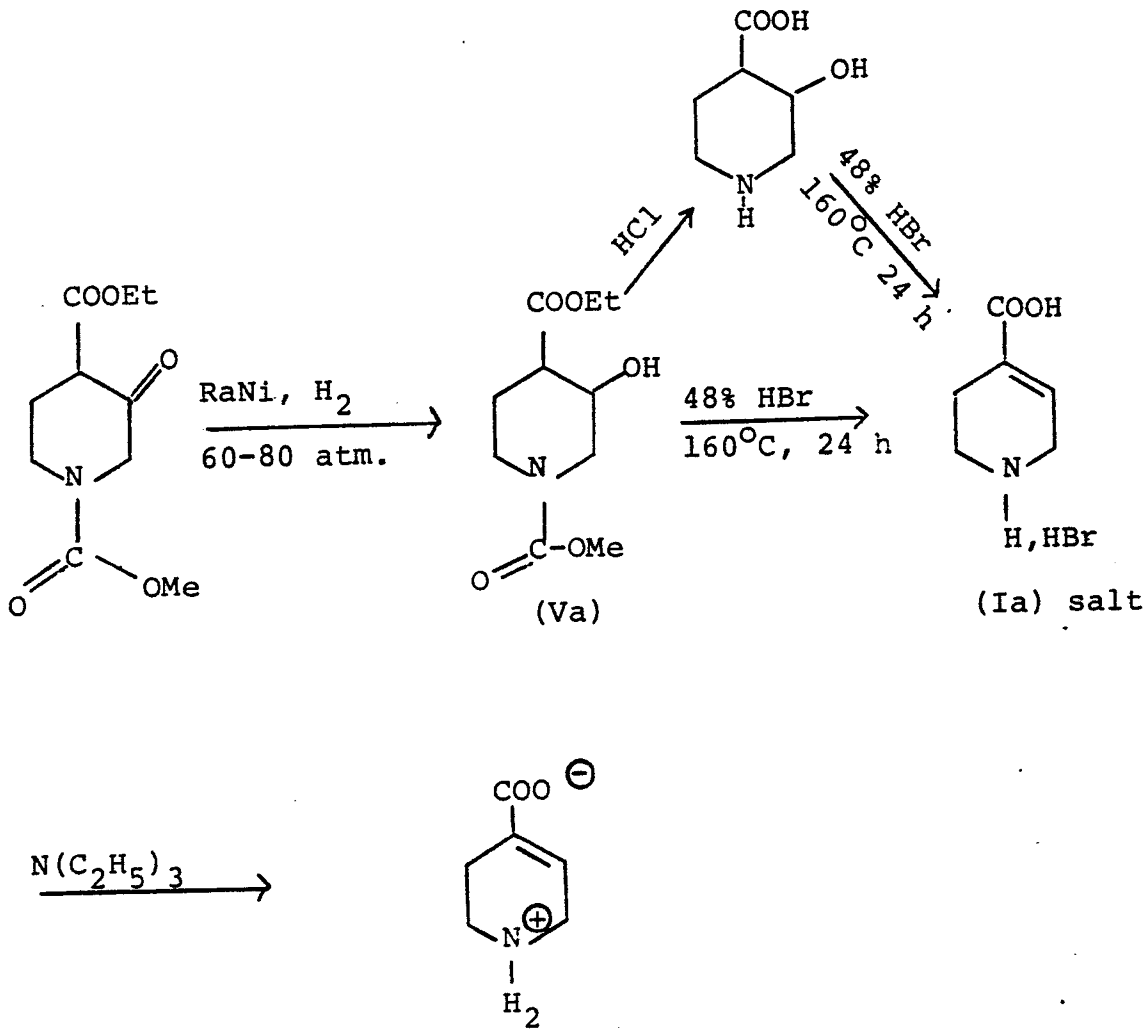


(Va)

(Ia) salt

In accordance with Reaction Scheme II, intermediates for the preparation of compound Ia are, on a generalized level, compounds of the general formula V

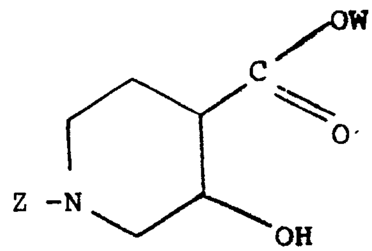

V

in which Z is hydrogen or an amino-protecting group removable by hydrolysis or hydrogenolysis, suitably a group R" as defined above or a trityl or formyl group, and W is hydrogen or a group readily removable, e.g. by hydrolysis, to yield the free carboxyl group, such as a group R' as defined above or tetrahydropyranyl.

In accordance with Reaction Scheme I, intermediates for the preparation of compound Ia are, on a generalized level, compounds of the general formula IV

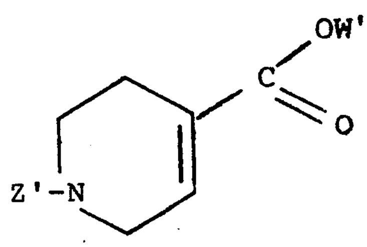

IV

in which Z' and W' have the same meaning as Z and W as defined above, with the proviso that at least one of Z' and W' is different from hydrogen.

For the preparation of compounds of the general formula I in which R' is lower alkyl, aryl, substituted aryl, aralkyl, or substituted aralkyl, compound Ia or compound IV in which W' is hydrogen may be esterified to introduce the desired group R', in accordance with any of the well known methods for the preparation of an ester of an amino acid, followed, by removal of any group Z' different from R" and if desired, removal of any group Z' which falls under the definitions of R". For the preparation of compounds of the general formula I in which R' is a group of the general formula III, a salt, e.g. a potassium salt of compound Ia or a compound of the general formula IV in which W' is hydrogen, is treated with a compound of the general formula

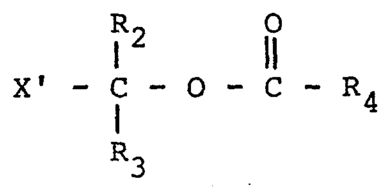

in which $R_2$, $R_3$ and $R_4$ are as defined above, in which X' is a halogen atom, e.g. chlorine, in the presence of an acid binding agent, e.g. potassium carbonate as used for the formation of the salt of Ia, and then, removing any group Z' different from R" and, if desired, any group Z' which falls under the definition of R".

The introduction of the group R" may be performed by manners known *per se*. Thus, for example, when R" is a group of the above formula II, the introduction may be performed by treatment with the appropriate formic acid ester of the general formula

$$X''-COOR_1$$

wherein X" is a leaving group, especially halogen or azido, etc., in the presence of an acid acceptor, for example an alkali carbonate. For example, the BOC-derivative can be made by means of tert.butylazidoformate. When R" is acetyl, a reactive derivative of acetic acid, e.g. acetyl chloride or acetanhydride may be used for the introduction of the group R".

More detailed aspects of the above processes of the invention appear from the claims.

The compounds of formula I and salts thereof may be formulated for administration in any convenient way by analogy with other pharmaceuticals.

Thus, the composition comprising the compounds of the invention may be in the form of pharmaceutical preparations, e.g. in solid, semisolid or liquid form, which contain the active compound of the invention in admixture with a pharmaceutical or inorganic carrier or excipient suitable for enteral or parenteral application. The active ingredient may, e.g., be formulated with the usual carriers for tablets, pellets, capsules, suppositories, solutions, emulsions, aqueous suspensions and other suitable administration forms. Examples of carriers are glucose, lactose, gum acacia, gelatin, mannitol, starch paste, magnesium trisilicate, talc, corn starch, keratin, colloidal silica, potato starch, urea, and other carriers suitable for use in manufacturing compositions in solid, semisolid, or liquid form, and in addition auxiliary, stabilizing, thickening, colouring, flavouring, and preservative agents can be contained in the composition of this invention.

The active compound is included in the compositions of the invention in an amount sufficient to produce the desired therapeutical effect upon administration. The dosage or therapeutically effective quantity of the compound varies and also depends upon the age and condition of each individual patient being treated.

A preferred tablet or capsule formation for oral administration contains 0.1 - 200 mg, preferably 1 - 100, especially 5 - 50, mg of a compound of formula I, or a salt thereof per unit dosage which may be administered 1 - 4 times per day or as a sustained release composition.

Injection preparations preferably contain 0.1 - 200 mg, preferably 1 - 100, especially 5 - 50, mg of a compound of formula I or a salt thereof per unit dosage. A preferred injected dose is about 0.5 to 2 ml.

The invention also relates to the use of the compounds of the general formula I, and salts thereof in medicaments for treating GABA system malfunction-related diseases, and a process of treating GABA system malfunction-related diseases in human beings by administering, to the human being, an effective dose of a compound of the general formula I, or a salt thereof.

In the above-mentioned compositions and the above-mentioned uses, it may be suitable or preferred to combine the compound of the general formula I or a salt thereof with minor tranquillizers such as benzodiazepines, or neuroleptics, for example butyrophenones

such as haloperidol, phenothiazines such as chloropromazine, thioxanthene, and the like. In such combinations, compositions and combined usages, the neuroleptics are suitably administered in their effective amounts or, in a preferred embodiment in lower amounts than the amounts in which they would be effective when used alone.

An interesting aspect of the invention is compound Ia as intermediate in the preparation of derivatives of compounds of the formula I wherein R" is different from hydrogen.

The invention is further illustrated by the below working examples. All compounds prepared according to the working examples have been subjected to elemental analysis for C, H, N and halogen, when present, and all agreed within $\pm$ 0.3% with the calculated values.

Example 1. (Reaction Scheme I).

Ethyl 1-ethoxycarbonyl-1,2,3,6-tetrahydroisonicotinate (IVa).

A solution of 1-methyl-1,2,3,6-tetrahydroisonicotinic acid hydrochloride (5.31 g; 30 mmol) in a solution of hydrogen chloride in ethanol (200 ml; 5%) was refluxed for 10 hours. Upon evaporation *in vacuo* the residue was dissolved in ice water (10 ml) and an iced aqueous solution of sodium hydrocide (15 ml; 30%) was added. The mixture was extracted with four 50 ml portions of methylene

chloride. The combined and dried ($K_2CO_3$) organic phases were evaporated to give 4.49 g of an oily TLC-pure product [$R_F$: 0.29; eluent: 1-butanol-water-acetic acid (4:1:1)]. A solution of this product (4.49 g) and ethyl chloroformate (25 ml) in dichloroethane (120 ml) was refluxed for 24 hours. The reaction mixture was filtered and evaporated in vacuo to give an oil. Ball-tube distillation at 95 Pa (oven temperature 160°C) gave IVa (5.52 g; 81%) as a colourless oil. IR (film): 2980 (m), 2930--2810 (several bands, m-w), 1700 (s), 1655 (m).

1,2,3,6-Tetrahydroisonicotinic acid hydrobromide (Ia salt).

A solution of ethyl 1-ethoxycarbonyl-1,2,3,6--tetrahydroisonicotinate (4.54 g; 20 mmol) in an aqueous solution of hydrogen bromide (30 ml; 48%) was refluxed for 2 hours. The solution was filtered and evaporated in vacuo to give a crystalline residue. Recrystallization (water--2-propanol-ether) gave Ia hydrobromide (2.45 g; 59%), m.p. 284--287°C (decomp.). IR (KBr): 3600--3300 (m), 3300--2850 (s), 2790 (s), 2700--2200 (several bands, m-w), 1710 (s), 1655 (s), 1630 (w), 1585 (m).

Using 8N hydrochloric acid instead of 48% aqueous hydrogen bromide and refluxing for 10 hours, the hydrochloride of Ia was obtained, m.p. 270--271.5°C (decomp.).

1,2,3,6-Tetrahydroisonicotinic acid zwitterion (Ia).

To a solution of 4.90 g of Ia hydrochloride in 45 ml of water a solution of triethylamine (3.18 g) in ethanol (90 ml) was added. The precipitate (2.5 g) was collected and recrystallized from water-ethanol yielding the title compound, m.p. 249--252°C. (decomp.).

Example 2. (Reaction Scheme II).

Ethyl 1-methoxycarbonyl-3-hydroxypiperidin-4-carboxylate (Va).

A solution of 15 g of ethyl 1-methoxycarbonyl-3-oxopiperidine--4-carboxylate (P. Krogsgaard-Larsen, Acta Chem. Scand., 1977, B31, 584) in 150 ml EtOH was mixed with Raney-nickel (from 10 g of Raney alloy) and hydrogenated at 10 MPa for 20 hours. The reaction mixture was filtered and evaporated to dryness *in vacuo*. An amount (4 g) of the residue was distilled yielding 3.4 g, b.p. at 0.2 mm Hg: 145--147°C.

1,2,3,6-Tetrahydroisonicotinic acid hydrobromide (Ia salt).

A mixture of the above hydroxy compound (Va) (0.5 g) and 1 ml of 48% aqueous hydrobromic acid was refluxed for 24 hours.

After cooling a compound crystallized. The compound was proved identical to the above prepared Ia hydrobromide by T.L.C., IR-spectra, and m.p.

An alternative route from Va to Ia is via 3-hydroxypiperidine--4-carboxylic acid hydrochloride as described below:

3-Hydroxypiperidine-4-carboxylic acid hydrochloride.

A mixture of Va (3.0 g) and 6 ml of conc. HCl in 5 ml of water was refluxed for 2 hours and evaporated to dryness *in vacuo*. The residue was recrystallized from water--glacial acetic acid--ether yielding 1.2 g (52%) of the title compound, m.p. 237--240°C.

Example 3.

Methyl 1,2,3,6-tetrahydroisonicotinate hydrochloride.

To a suspension of 4.91 g of Ia hydrochloride in 250 ml of methanol kept at 40°C was added a stream of hydrochloric acid (gas) for 1 hour. The solution obtained was left overnight and evaporated, yielding 4.25 g (80%) of the title compound, m.p.: 158--159°C. Recrystallization from acetonitrile raised the m.p. to 160--161°C.

Example 4.

1-[illegible]hydroisonicotinic acid.

To a suspension of Ia hydrochloride (0.49 g) in 10 ml of pyridine was added 0.57 ml of acetic acid anhydride. The mixture was stirred at 20°C for 1 hour and then at 70°C for 1 hour. The reaction mixture was evaporated in vacuo and water (10 ml) was added to the residue followed by 4N HCl to pH = 2. The aqueous solution was extracted with four 10 ml portions of $CHCl_3$. The combined chloroform layers were washed with 10 ml of water, dried and evaporated in vacuo. The residue was treated with 10 ml of ether and 0.28 g (55%) was obtained. Recrystallizations from ethyl acetate gave the title compound, m.p. 177--182°C.

Example 5.

1-tert.Butyloxycarbonyl-1,2,3,6-tetrahydroisonicotinic acid.

To a solution of Ia hydrochloride (1.64 g) in water (15 ml) was added triethylamine (5.6 ml) followed by a solution of tert.-butyl azidoformate (1.7 ml) in dioxane (15 ml). The mixture was stirred at 20°C for 15 hours and the dioxane was evaporated in vacuo. The aqueous solution was washed with two 20 ml portions of ether, cooled to 3°C and acidified with 1N HCl. The precipitate (1.95 g, 86%) was collected and recrystallized from toluene yielding the title compound, m.p. 148--150°C.

Example 6.

1-Benzyloxycarbonyl-1,2,3,6-tetrahydroisonicotinic acid.

A solution of Ia hydrochloride (1.63 g) in 50 ml of 2N NaOH was cooled to 0°C. To this solution were added simultaneously benzyl chloroformate (7.8 ml) and 2N NaOH (27.5 ml) during 10 min. The mixture was stirred at 0°C for 1 hour, and washed with 25 ml of ether. The aqueous phase was cooled and acidified with 4N HCl. The white precipitate was collected and recrystallized from a mixture of toluene and petroleum ether yielding 8.7 g (67%) of the title compound, m.p. 106--108°C (decomp.).

Example 7.

Pivaloyloxymethyl 1-tert.butyloxycarbonyl-1,2,3,6-tetrahydroisonicotinate.

To a solution of 1-tert.butyloxycarbonyl-1,2,3,6-tetrahydroisonicotinic acid (1.36 g) in 85 ml of acetone potassium carbonate (1.24 g) was added. The mixture was stirred for 10 minutes and pivaloyloxymethyl chloride (0.96 ml) was added dropwise. The mixture was stirred for 2 hours and then refluxed for 7 hours. The cooled reaction mixture was filtered and the filtrate was evaporated *in vacuo*. The residue was extracted with 30 ml of ether and the ether was evaporated leaving the title compound as a colourless oil (1.56 g, 76%).

Example 8.

Pivaloyloxymethyl 1,2,3,6-tetrahydroisonicotinate hydrobromide.

To a solution of pivaloyloxymethyl 1-tert.butyloxycarbonyl--1,2,3,6-tetrahydroisonicotinate (1.45 g) in 10 ml of ethyl acetate was added a 48% solution of HBr in glacial acetic acid (1.2 ml). After 5 minutes at 20°C ether (80 ml) was added in small portions. The precipitate was collected and recrystallized from ethyl acetate-ether leaving 549 mg (40%) of the title compound, m.p. 70--73°C.

Example 9.

Butyl 1,2,3,6-tetrahydroisonicotinate hydrobromide.

Butyl 1-tert.butyloxycarbonyl-1,2,3,6-tetrahydroisonicotinate was obtained in the same manner as described in Example 7 using butyl bromide instead of pivaloyloxymethyl chloride. Treating the compound obtained in the same way as described in Example 8 yielded the title compound, m.p. 105--106°C (from methanol--ether).

Example 10.

Methyl 1-triphenylmethyl-1,2,3,6-tetrahydroisonicotinate.

To a solution of methyl 1,2,3,6-tetrahydroisonicotinate hydrobromide (1.1 g) in 10 ml of chloroform were added 1.53 ml of triethylamine and 1.39 g of triphenyl chloromethane. The mixture was stirred at 20°C for 6 hours. The reaction mixture was washed with two 10 ml portions of water, dried, and evaporated *in vacuo*. The residue was triturated with warm methanol leaving 1.60 g (83%). One recrystallization from ethanol yielded the title compound, m.p. 172--174°C.

Example 11.

1-Triphenylmethyl-1,2,3,6-tetrahydroisonicotinic acid.

A suspension of the methyl ester of the title compound (1.5 g) in a solution of 0.24 g potassium hydroxide in 10 ml of ethanol was refluxed for 17 hours. Water (40 ml) was added and the mixture was acidified with a 50% acetic acid (5 ml). The precipitate (1.45 g) was collected and recrystallized from chloroform-petroleum ether yielding the title compound, m.p. 161--165°C (decomp.).

Example 12.

Pivaloyloxymethyl 1,2,3,6-tetrahydroisonicotinate hydrobromide.

Reacting the potassium salt of 1-triphenylmethyl-1,2,3,6-tetrahydroisonicotinic acid with pivaloyloxymethyl chloride in the same manner

BAD ORIGINAL

as described in Example 7 yielded pivaloyloxymethyl 1-triphenylmethyl-1,2,3,6-tetrahydroisonicotinate as an oil. Treating this oil with hydrobromic acid in glacial acetic acid in the same way as described in Example 8 yielded a compound, which was proved identical to the compound prepared in Example 8 by T.L.C. and m.p.

Claims:

1. Compounds of the general formula I

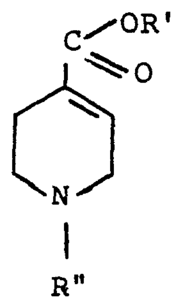

I

in which R" is hydrogen, acetyl or a group of the general formula II

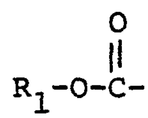

II

wherein $R_1$ is $C_{1-8}$ alkyl; phenyl; phenyl substituted in the 4-position with halogen, lower alkoxy, or lower alkyl; or phenylalkyl such as benzyl or phenylethyl in which the phenyl group may be substituted in the 4-position with halogen, lower alkoxy, or lower alkyl; and R' is hydrogen; $C_{1-8}$ alkyl; phenyl; phenyl substituted in the 4-position with halogen, lower alkoxy, lower alkyl or hydroxy; phenylalkyl such as benzyl or phenylethyl; or phenylalkyl substituted in the 4-position of the phenyl moiety with halogen, lower alkoxy, lower alkyl or hydroxy; or indanyl; or R' is a group of the general formula III

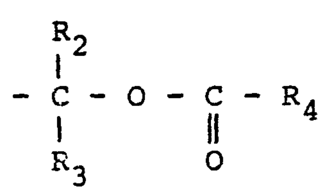

III

wherein $R_2$ and $R_3$ are the same or different and each designate hydrogen; $C_{1-6}$ alkyl; or phenylalkyl such as benzyl or phenylethyl; and $R_4$ designates $C_{1-8}$ alkyl; phenyl; phenyl substituted in the 4-position with halogen, lower alkoxy or lower alkyl; or phenylalkyl such as benzyl or phenylethyl; and salts thereof, with the proviso that when R" is hydrogen, R' is different from ethyl.

2. A compound according to claim 1, characterized in that it is 1,2,3,6-tetrahydroisonicotinic acid, and salts thereof.

3. Compounds according to claim 1, in which R" is hydrogen, and R' is a group of the general formula III as defined in claim 1; and salts thereof.

4. A compound according to claim 3, characterized in that it is pivaloyloxymethyl 1,2,3,6-tetrahydroisonicotinate, and salts thereof.

5. Compounds of the general formula V

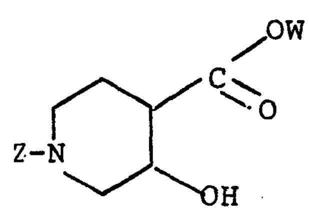

V

in which Z is hydrogen or an amino-protecting group removable by hydrolysis or hydrogenolysis, and W is hydrogen or a group readily removable to yield the free carboxyl group, and salts thereof.

0000167

6. Compounds of the general formula IV

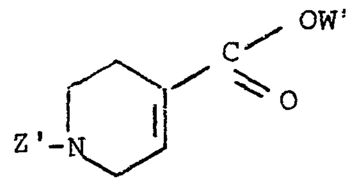

IV

in which Z' and W' have the same meaning as Z and W as defined in claim 5, with the proviso that at least one of Z' and W' is different from hydrogen, and salts thereof.

7. A pharmaceutical composition comprising as active ingredient a compound of the general formula I'

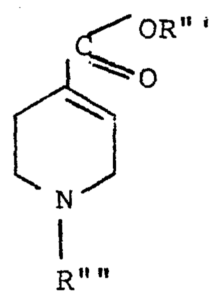

I'

in which R"" is hydrogen, acetyl or a group of the general formula II

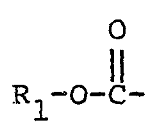

II

wherein $R_1$ is as defined in claim 1; and R"' is hydrogen; $C_{1-8}$ alkyl, phenyl; phenyl substituted in the 4-position with halogen, lower alkoxy, lower alkyl or hydroxy; phenylalkyl such as benzyl or phenylethyl; or phenylalkyl substituted in the 4-position

BAD ORIGINAL

of the phenyl moiety with halogen, lower alkoxy, lower alkyl or hydroxy; or indanyl; or R"' is a group of the general formula III

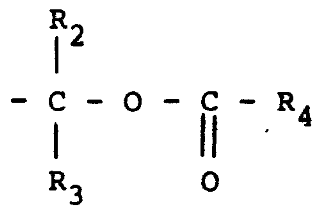

III

wherein $R_2$ and $R_3$ and $R_4$ are as defined in claim 1; or a salt thereof together with a pharmaceutical carrier or excipient.

8. A pharmaceutical composition according to claim 7 which additionally contains a minor tranquillizer or a neuroleptic.

9. A process for preparing compounds of the general formula I

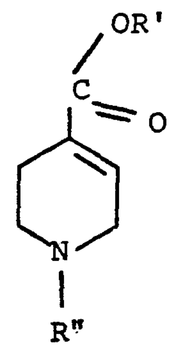

I

in which R" is hydrogen, acetyl or a group of the general formula II

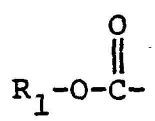

II

wherein $R_1$ is $C_{1-8}$ alkyl; phenyl; phenyl substituted in the 4-position with halogen, lower alkoxy, or lower alkyl; or phenylalkyl such as benzyl or phenylethyl in which the phenyl group may be substituted in the 4-position with halogen, lower alkoxy, or lower alkyl; and R' is hydrogen; $C_{1-8}$ alkyl; phenyl; phenyl substituted in 4-position with halogen, lower alkoxy, lower alkyl or hydroxy; phenylalkyl such as benzyl or phenylethyl; or phenylalkyl substituted in the 4-position of the phenyl moiety with halogen, lower alkoxy, lower alkyl or hydroxy; or indanyl; or R' is a group of the general formula III

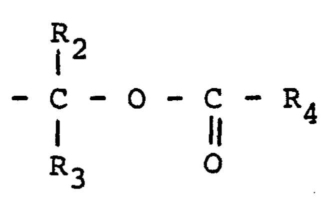

III

wherein $R_2$ and $R_3$ are the same or different and each designate hydrogen; $C_{1-6}$ alkyl; or phenylalkyl such as benzyl or phenylethyl; and $R_4$ designates $C_{1-8}$ alkyl; phenyl; phenyl substituted in the 4-position with halogen, lower alkoxy or lower alkyl; or phenylalkyl such as benzyl or phenylethyl; and salts thereof, with the proviso that when R" is hydrogen, R' is different from ethyl, comprising

a) reacting a compound of the general formula VI

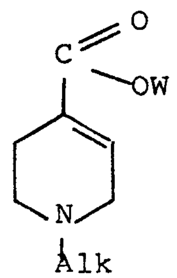

VI

in which Alk is a lower alkyl group and W is hydrogen or group removable to yield the free carboxy group, with a formic acid ester of the general formula

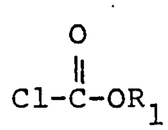

in which $R_1$ is as defined above, to form a compound of the general formula

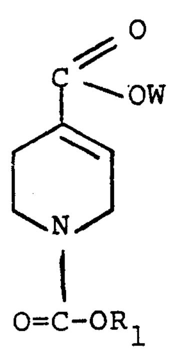

removing any group W different from R'; if desired, removing the group $R_1-O-\overset{O}{\overset{\|}{C}}-$, and, if desired, removing any group W different from hydrogen, if desired, converting a resulting compound of formula Ia

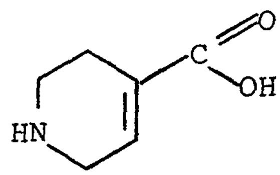

Ia

if obtained as a salt thereof, into the zwitterion form thereof by treatment with a base or into another salt, and, if desired, converting the compound Ia into a compound VII

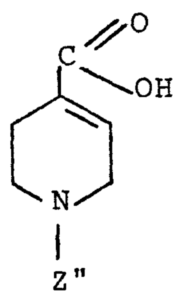

VII

in which Z" is a group R" as defined above or a trityl or formyl group, by reacting compound Ia with a formic acid ester of the general formula

$$X''-COOR_1$$

in which $R_1$ is as defined above, and X" is a leaving group, or with trityl chloride, or with the mixed anhydride of acetic acid and formic acid, or with a reactive acetic acid derivative;

if desired, esterifying the carboxy group in the resulting compound VII or a salt thereof, to introduce a group R' which is lower alkyl, aryl, substituted aryl, or substituted aralkyl, or treating a salt of compound VII with a compound of the general formula

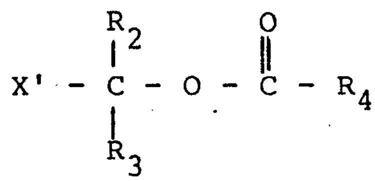

in which $R_2$, $R_3$ and $R_4$ are as defined above, and X' is a halogen atom, in the presence of an acid binding agent, and thereafter removing any group Z" different from R", and if desired, any group Z", or

b) subjecting a compound of the general formula IV

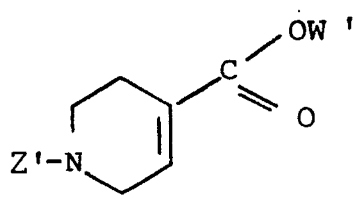

IV

in which Z' and W' have the same meanings as defined in claim 6, to removal of the group W' and/or Z', with the proviso that any group W' or Z' different from R' or R", respectively, is removed, or

c) subjecting a compound of the general formula V

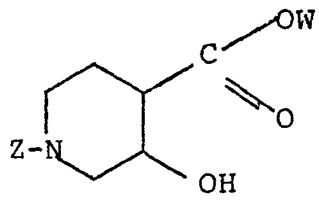

V

in which Z and W have the same meanings as defined in claim 5, to dehydratisation, or

d) converting the compound Ia into a compound VII as defined above by reacting compound Ia with a formic acid ester of the general formula

$$X"-COOR_1$$

in which $R_1$ is as defined above, and X" is a leaving group, or with trityl chloride, or with the mixed anhydride of acetic acid and formic acid, or with a reactive acetic acid derivative; if desired, esterifying the carboxy group in the resulting compound VII or a salt thereof, to introduce a group R' which is lower alkyl, aryl, substituted aryl, or substituted aralkyl, or treating a salt of compound VII with a compound of the general formula

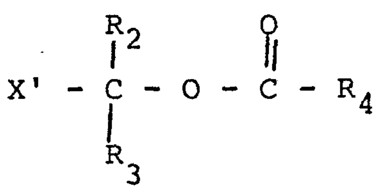

in which $R_2$, $R_3$ and $R_4$ are as defined above, and X' is a halogen atom, in the presence of an acid binding agent, and thereafter removing any group Z" different from R", and if desired, any group Z", or

e) esterifying the carboxy group in compound Ia or a salt thereof, to introduce a group R' which is lower alkyl, aryl, substituted aryl, or substituted aralkyl, or treating a salt of compound Ia with a compound of the general formula

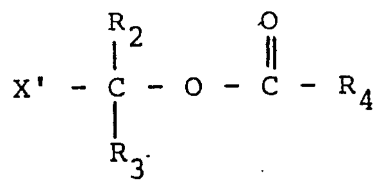

in which $R_2$, $R_3$ and $R_4$ are as defined above, and X' is a halogen atom, in the presence of an acid binding agent.

European Patent Office

# EUROPEAN SEARCH REPORT

0000167

Application number

EP 78 10 0190

## DOCUMENTS CONSIDERED TO BE RELEVANT

| Category | Citation of document with indication, where appropriate, of relevant passages | Relevant to claim |
|---|---|---|
| X | BERICHTE DER DEUTSCHEN CHEMISCHEN GESELLSCHAFT 51 (1918) 806-820.<br>* Pages 809, 820 *<br>-- | 1,2 |
| | DE - A - 2 221 770 (BAYER A.G.)<br>* Page 6, examples 1a), b) *<br>-- | 6 |
| | CHEMICAL ABSTRACTS, 59 (1963) 558b<br>T. TSUKAMOTO et al.: "Synthesis of 1-methyl-1,2,5,6-tetrahydro-3 (and 4) -pyridinecarboxylates".<br>& YAKUGAKU ZASSHI 82, 1317-19 (1962)<br>-- | 1 |
| X | CHEMICAL ABSTRACTS 52 (1958)13724h-13725c<br>M. FERLES: "Pyridine series III "Electrolytic and lithium aluminum hydride reductions of quaternary pyridinium salts"<br>& Chem. Listy 52, 674-81 (1958)<br>* 13725c *<br>-- | 1 |
| | DK 62 791(LØVENS KEMISKE FABRIK VED. A. KONGSTED.)<br>& CHEMICAL ABSTRACTS 40 (1946), 4181[8].<br>-- | 1 |
| | CHEMICAL ABSTRACTS 13 (1919) pages 2023<br>& Z. Physiol. Chem. 104,48-53 (1918)<br>-- | 1 |
| | CHEMICAL ABSTRACTS 8 (1914) page 947<br>& Z. Physiol. Chem. 85, 372-91.<br>-- | 1 |

./.

CLASSIFICATION OF THE APPLICATION (Int. Cl.²)

C 07 D 211/78
C 07 D 211/62
A 61 K 31/435

TECHNICAL FIELDS SEARCHED (Int.Cl.²)

C 07 D 211/78

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 25-08-1978 | VAN BIJLEN |